# EUROPEAN PATENT SPECIFICATION

(11) **EP 4 271 452 B1**
(45) Date of publication and mention of the grant of the patent: **19.02.2025**
(21) Application number: 22701268.9
(22) Date of filing: 01.02.2022
(51) Int. Cl.: A61M 15/08, A61M 3/02, A61H 35/04, A61H 33/04

(54) **DISPOSABLE NASAL LIQUID DISPENSER DEVICE**
WEGWERFBARE NASALE FLÜSSIGKEITSABGABEVORRICHTUNG
DISPOSITIF DE DISTRIBUTION DE LIQUIDE NASAL JETABLE

(30) Priority: 10.02.2021 EP 21156429
(43) Date of publication of application: 08.11.2023
(73) Proprietor: NAOUM, George, 11475 Gkizi Attikis (GR)
(72) Inventor: NAOUM, George, 11475 Gkizi Attikis (GR)
(74) Representative: Araujo, Daniel
(86) International application number: PCT/EP2022/052285
(87) International publication number: WO 2022/171483

(56) References cited:
- WO-A1-93/11818
- DE-A1- 10 006 026
- DE-C- 203 358
- FR-A1- 3 016 125
- FR-A1- 3 092 991
- US-A1- 2010 152 653
- US-A1- 2015 045 751

## Description

### Field of the invention

The present invention relates to a disposable nasal liquid dispenser device for administering a controlled quantity of a liquid solution to the nasal cavities and / or paranasal sinuses of a user. The device is intended to be used for the cleaning and / or disinfection of nasal cavities and paranasal sinuses and / or for the treatment of nasal and / or sinus disorders such as sinus inflammation, including nasal congestion.

### Background

Sinus and nasal congestion or rhinitis, involves the blockage of one or more of the paranasal sinus passageways in the skull. The paranasal sinuses are air-filled cavities within the facial skeleton. Each paranasal sinus is contiguous with a nasal cavity and connects with it via small orifices called ostia. The blockage of the ostia may result from inflammation and swelling of nasal tissues or the presence of excess mucus. When inflammation occurs, normal drainage of mucus from within the sinuses is disrupted, and sinusitis may occur.

Described paranasal sinuses are frontalis sinuses, ethmoid sinuses, sphenoid sinuses and maxillary sinuses.

While acute nasal congestion is frequently caused by acute infections, chronic nasal congestion usually results from exposure to irritants such as tobacco smoke, food allergens, inhaled allergens, or foreign matter within the nasal cavity. So, maintenance of clear and healthy nasal cavities and paranasal sinuses of humans is part of proper medical, family and / or personal care.

Further, the respiratory system is subject to continuous risks of viral diseases and microbial infections, mainly during the winter. SARS-Cov2 infection mainly begins in the nasopharyngeal area, it is incubated there for a period of time and then it gains entrance to the rest of the respiratory tract^{1,2}. Hypertonic saline exposure of human cells like epithelial, mucosal and immunity cells in the upper respiratory system increases their antiviral activity against many respiratory pathogens including previously known coronaviruses and SARS-Cov2. Specifically, hypertonic saline solution promotes innate immunity responses in various ways, such as inducing depolarization of the epithelial cells putting them in a low energy state, thus decreasing the ability of the virus to replicate³ and / or it induces increased production of hypochlorite ions via myeloperoxidase dependent reaction due to increased chloride ions concentration⁴. This increase in antiviral innate immunity has been shown clinically in upper respiratory infection including other coronaviruses where the use of hypertonic saline has reduced both the duration of disease and the need for use of other medications⁵.

Traditional techniques to clean the nasal cavities and to treat nasal congestion include techniques of nasal irrigation and use of decongestant sprays which cause vasoconstriction of the nasal cavity mucosa reducing inflammation.

Decongestant sprays rapidly reduce edema mainly in the nasal cavity mucosa and make breathing through the nose easier. However, these sprays do not reach the whole mucous membrane surface of the nasal cavities and the paranasal sinuses. It is needed to reach these parts of the mucosa to treat illnesses, e.g. sinusitis, which cannot be cured by current limited local treatment.

Another way of administering liquid solutions into the nasal cavities is by allowing a solution to flow through a tightening olive, which fits to the nostril, on its upper side and can be attached to some kind of container or tube on its bottom. Although the olive fits the nostril, it is not possible to fill the nose and let the solution reach the upper and inner parts of the nose, as the solution runs back into the pharynx along the bottom of the cavities.

Researchers and medical device manufacturers, acknowledging the great benefits to humans of having a healthy respiratory system, continuously try to find ways to inject therapeutic solutions to the paranasal sinuses. Many existing devices use various techniques but only partially achieve cleaning of the nostrils from excretions and do not succeed in injecting therapeutic solution to the paranasal sinuses in a controlled way.

For example, the device described in US 2014/0121592 provide liquid in the nasal cavities, allowing for a quick cleaning or short-term treatment of the nasal cavities. However, the infected fluid returns to the container from the same spout, probably passing some infected material to the clean liquid. This short term period leads to a temporary solution.

In addition, the device of WO 2013/072856 has to be connected to a source of compressed air in order to provide the fluid in the nostril. If the user was to receive a treatment in the paranasal cavities, the use of such a device would either cause the user to choke and/or to feel highly uncomfortable, stressed, while injury could be provoked to upper respiratory system including ears via eustcahian tubes.

In addition, WO 99/26684 describes a device designed for delivering a constant controlled flow of liquid or many equal doses of liquid to the nasal cavities. However, even this device, is used for rinsing the nasal cavities and does not allow for the liquid to enter and remain in the nasal cavities and paranasal sinuses and thus does not face the root of the problem. Although this device is supposed to provide liquid in a more controllable way, if it is to be used with a delivering probe, like the one described in US 2017/0340869 which blocks the nostrils in order for the liquid to reach the paranasal cavities, the continuous flow would probably cause choking to the user.

WO 93/11818 A1 discloses a dispenser for dispensing a dosage or a powdery or fluid drug from two independent chambers arranged within a piston, wherein the ends of each chamber are sealed by respective breakable membranes. The dispenser further comprises a cylindrical housing closed by a plunger which is displaceable into the cylinder. The dispenser further comprises two outlet pipes, each of which is aligned with and connected to only one of the chambers, wherein each outlet pipe comprises a respective penetrator configured to penetrate a membrane of the respective chamber upon displacement of the plunger into the cylinder, thereby delivering the content of the chamber.

DE 100 06 026 A1 describes dispenser for administering gaseous or liquid media comprising a housing for accommodation of the media, a pressure generator device, and two dispensing sections whose end sections are respectively configured as conical application nozzles. The disclosed dispenser is formed from a dimensionally stable plastic body which extends into the dispensing sections. The distance between the two dispensing sections is adapted to the distance between the nostrils of the user.

DE 203 358 C discloses a dispenser for injecting a small and constant amount of liquid into the nostrils of a person. The dispenser comprises liquid container, a spray ball configured to pump air upon squeezing, a single output tube having a first end configured as a fork-shaped nozzle for delivering the amount of liquid into the nostrils, and a rotating dosing passage. The rotating dosing passage is connected to an actuator to manually alternate between two operating positions: a fist position in which the passage communicates with the liquid container to receive the amount of liquid to be dispensed while the rotating dosing passage blocks the output tube; and a second position in which the rotating dosing passage is aligned with the output tube, thereby enabling the delivery of the amount of liquid through the fork-shaped nozzle upon squeezing of the spray ball.

Consequently, new devices for dispensing a liquid solution, which make it possible to fill the nasal cavities with a liquid solution, thereby reaching the paranasal sinuses and covering the whole nasal mucous membrane surface while avoiding the solution to run back into the pharynx, are desirable.

### Summary

The proposed device hereof may provide for cleaning and / or disinfecting the nasal cavities and paranasal sinuses and may also achieve injection of a liquid solution to the paranasal sinuses for the treatment of sinus infections.

To this aim, according to an example, the nasal liquid dispenser device for dispensing a controlled quantity of a liquid solution to nasal cavities and / or the paranasal sinuses of a user, is comprising:
- a liquid solution holding cylinder having a distal and a proximal end and containing a quantity of liquid solution to be dispensed; the cylinder comprising two cannulas arranged at its distal end for dispensing the liquid solution to both nasal cavities simultaneously;
- two resilient nostril plugs to seal the nostrils upon application therein, having a distal and proximal end; the plugs comprising coupling means configured to be mounted on the cannulas of the liquid solution holding cylinder and connected to a discharge orifice arranged at the distal end of the nostril plugs;
- a piston mounted in the cylinder and comprising elastic piston seals to offer sealing on the liquid solution holding cylinder; the piston is designed to slide in an axial direction relative to said cylinder; and
- an actuator mounted with the piston and configured to move the piston along the inside of the cylinder upon manual actuation of a user.

The device is formed in such a way to administer a liquid solution into the sinus cavity, sinus ostium, and / or nasal passage to treat a paranasal sinus condition.

The liquid solution holding cylinder may comprise a saline solution for bathing the nasal and sinus cavities to wash away encrusted mucous, irritants, and foreign particles for the purpose of improving airflow and relieving nasal congestion. However, the cylinder may also contain other liquid solutions, such as medicinal solutions, e.g. antibiotic solutions, for the upper respiratory system or liquids containing extracts of herbal plants which may also or alternatively be beneficial for the upper respiratory system.

Many existing devices use various techniques but only partially achieve a cleaning of the nostrils and do not succeed in injecting, in a controlled manner, therapeutic solutions to the paranasal sinuses.

The filling of the paranasal sinuses may be achieved with the use of the proposed device. The liquid solution is provided to the nasal cavities through the resilient nostril plugs, designed to lock into the nostrils, generating an airtight seal between the plugs and the nostrils, and triggering a swallow reflex that raises the soft palate, sealing off the nasal cavity and exerting pressure to the liquid that may then find an escape route through the paranasal sinus passageways and into the paranasal sinuses.

The shape, form and size of the nostril plugs is advantageous for the proper functioning of the nostril plugs. The nostril plugs are designed to be bell-shaped to follow the shape of the nose and lock into the inner cavity as soon as they are pressed in the nose. A further advantageous aspect of the nostril plugs in their design is the angled arrangement of the same to correspond with the angled distribution of the nostrils of a user, so that they offer perfect adaptation and ergonomics to the human nose but also offer comfort and safety in the nasal cavity.

The use of such a system makes easier the delivery of liquid to the paranasal sinuses, since the nostril plugs are fitted in the nostrils blocking them and not allowing the fluid to exit the nose. Treatment or cleaning of the paranasal sinuses cannot be delivered simply by rinsing the nasal cavities, and requires an advanced quantity of fluid to be held in the nasal cavities, which is succeeded by prohibiting the fluid to exit the nasal cavities until the treatment is completed.

By holding, and gently pressing the actuator, the piston slides into the cylinder, thus dispensing the liquid solution to the nasal cavities. The quantity and rate of pouring of the liquid solution may be regulated by the user. Also, due to the fact that the quantity of fluid in the cylinder corresponds to the quantity of fluid that is required to fill both the nasal and the paranasal cavities of a user, the user knows that when the fluid is over, he / she has received the whole treatment.

The shape and design of the piston and the actuator does not allow the user to return the piston to its original position. In case the user tries to refill the device with a solution, it will not be possible because when the actuator is moved in the opposite direction, apart from the cylinder, it will detach from the piston and the device will cease to function. The actuator of the piston of the device is moveable with the pressure of the finger at the extreme point of the container by dragging the piston together inwards. The two components, the actuator and the piston, are not permanently attached (e.g. glued) together so that during use the actuator pushes the piston inside the chamber but when the actuator tries to return it detaches from the piston and the piston cannot be returned to its original position (the one before pressure was applied). With the complete movement of the actuator and the piston, both components are inside the chamber. At this position the user is not able to hold with his fingers the actuator or try to return the piston to its original position. All of the above are intended to prevent the user from refilling the device with other solutions or to reuse the device in general, e.g. with third-party or unauthorised solutions.

The device is designed as a portable single-use device with a disposable configuration, assembled as a hand-held device for convenient lifting and disposal against the user's nostrils, which is easy to use and allows for self-administration.

### Brief description of the drawings

Non-limiting examples of the present disclosure will be described in the following, with reference to the appended drawings, in which:
Fig, 1 is an exploded view of a device for dispensing a liquid solution into the nasal cavities and / or paranasal sinuses according to an example hereof;
Fig. 2 is a partial view of the inside of the cylinder of the device corresponding to Fig. 1, for showing internal elements;
Fig. 3 shows a detail of the upper part of the cylinder and the nostril plugs of the device corresponding to Fig. 1;
Fig. 4 shows the arrangement of the nostril plugs relative to the nose of a user and relative to each other.

### Detailed description

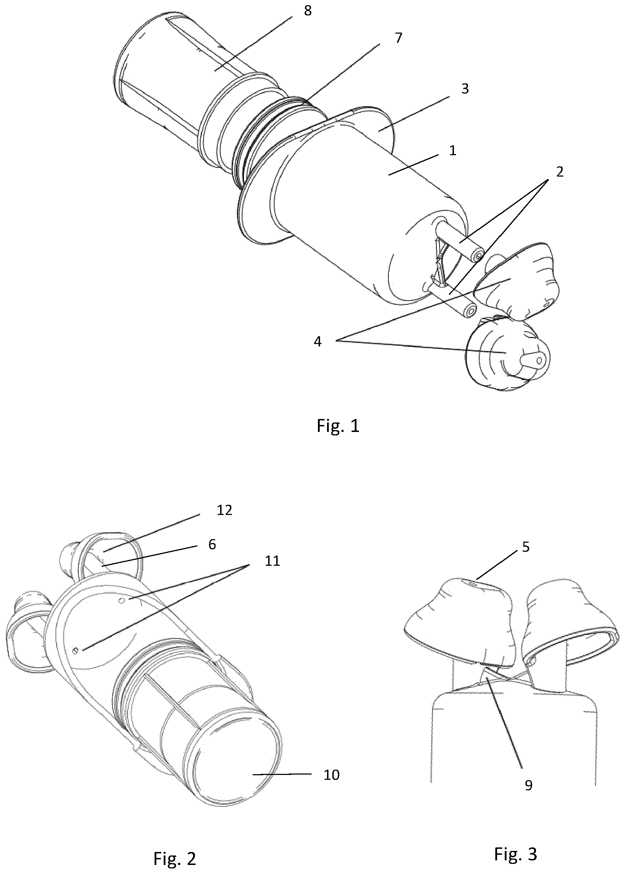

Fig. 1 is an exploded view of a disposable nasal liquid dispenser device for administering a liquid solution to the nasal cavities and / or paranasal sinuses of a user according to the present invention. The device includes a liquid solution holding cylinder 1 having a distal and a proximal end and containing a quantity of liquid solution to be dispensed. The cylinder 1 comprises two cannulas 2 arranged at its distal end for dispensing the liquid solution simultaneously and in a controlled manner, in equal and fixed doses, to both nasal cavities of a user.

The liquid solution holding cylinder 1 has two radially extending shoulders 3 arranged at its proximal end designed for holding the device during use.

The device further includes two resilient nostril plugs 4 having a distal and a proximal end and designed to seal the nostrils upon application therein.

As shown in Figs 2 and 3, the nostril plugs 4 are designed to be soft and comfortable, having a hollow interior 12 comprising coupling means 6 configured to be mounted on the cannulas 2 and said coupling means 6 connected to a discharge orifice 5 arranged at the distal end of the nostril plugs 4.

In a preferred embodiment, the nostril plugs 4 are made from LDPE low density polyethylene.

The nostril plugs 4 are bell-shaped to lock into the nose.

In an embodiment, the device is provided with different size nostril plugs to suit all users.

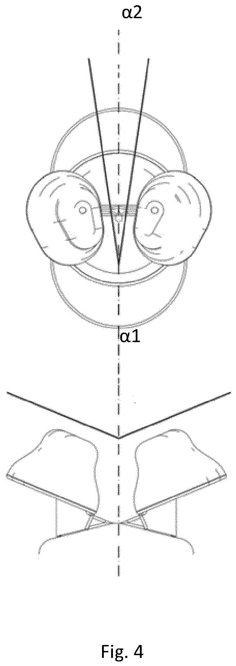

An important part of the nostril plugs 4 in their design is the arrangement of the same with regards to the nose of a user to offer perfect adaptation and ergonomics to the human nose. The nostril plugs are designed to rest in an angle α1 relative to the nose of a user of between 130° and 140°, when mounted on the cannulas 2, as shown in Fig. 4.

In a preferred embodiment the angle a1 is 135 degrees.

The nostril plugs 4 have a relative angle a2 between them of between 15 and 20 degrees.

The nostril plugs 4 may be affixed to the cylinder 1 by means of a snap-fit system 9. The snap-fit system is designed for mating together two rods arranged at the distal end of the cylinder 1 with a groove arranged in each of the nostril plugs coupling means 6, respectively.

A piston 7 is mounted in the proximal end of the cylinder 1 and designed to slide in an axial direction relative to said cylinder. The piston comprises elastic piston seals to offer sealing and insulation of the liquid solution contained in the liquid solution holding cylinder 1 till the moment of its use.

An actuator 8 is mounted with the piston 7 and configured to move the piston along the inside of the cylinder 1 upon manual actuation of a user on actuation surface 10. Said actuation surface 10 is sufficiently wide to support an average human thumb, by which the dispenser is actuated.

The actuator 8 is designed to detach from the piston 7 when moved in an opposite direction with regard to the liquid solution holding cylinder 1, preventing the user from returning the piston to its original position.

By holding, and gently pressing the actuator surface 10, the piston 7 slides into the cylinder 1, thus causing the liquid solution to flow through two holes 11, arranged in the inside of the cylinder 1, into the cannulas 2 and through the discharge orifices 5 of the nostril plugs into both nasal cavities of a user simultaneously. The quantity and rate of pouring is regulated by the user.

During use, the user inclines the head forward to avoid passage of the liquid to the pharynx. Then, liquid is administered by pressing the actuator surface multiple times until the level of the provided solution reaches slowly to the plane of the soft palate. Then the user may try to swallow or the reflex of swallowing may be triggered. The reflex movement of swallowing may be performed due to the stimulation of the soft palate by the intranasal liquid or therapeutic solution even though there is no bolus in the mouth for swallowing. To perform the swallowing reflex the soft palate may move upwards and frontwards. The available space may thus be reduced and the level of the liquid or solution may be raised and cover the orifices of the ducts of the paranasal sinuses. At the same time, the forward movement of the soft palate may push air towards the surface of the solution. Consequently, pressure may be exerted on the solution to push it towards the nostrils' openings. Because the nostrils' openings are blocked by the intranasal parts of the device, the solution may seek escape through the orifices of the ducts of the paranasal sinuses. In repetition of the swallowing movement the liquid may find an escape route through the paranasal sinuses' ducts, while the user may press the actuator surface of the device to slowly replenish the quantity of the solution so that the level of the solution remains at the level of the soft palate and maintains the stimulation of the soft palate.

The perfectly tolerable use of the device may give the user the possibility to use the therapeutic process with ease, even when at work or while sitting on a couch (e.g. while watching TV), for as long as a user wants, providing an effective method. The friendliness of a prolonged therapeutic use provides the necessary time for the therapeutic solution to act during a necessary time to maximize the therapeutic result in the nasal and paranasal chambers. The time may range between five (5) and fifteen (15) minutes. However, in some cases even longer periods of half an hour may be beneficial so that the therapeutic effect of the liquid may take place.

The end result may provide an effective solution desired and long sought after by the medical community and users. It may be made possible by the combination of characteristics of the proposed devices and/or methods. That is, the device may provide a complete sealing of the nostrils, thus provoking the swallowing reflex when sufficient liquid is introduced in the nostrils. Moreover, proper obstruction of both nostrils provided by the use of the proposed devices and/or methods, may allow the solution to remain in the nostrils and in the paranasal sinuses for as long as the user may wish, thereby providing the necessary time so that the therapeutic and/or cell-regenerative and/or healing properties of the medicine or of the hypertonic liquid (e.g. saline or sea water) or of the extracts of aromatic plants to give results.

Therefore, the therapeutic result for the user or patient may depend on the proper application of the process that is provided by the proposed devices. It is also mentioned that an application of the solution to the nostrils may be performed in a soft and totally controllable manner and in small doses. This may in some applications be achieved by the device construction that may provide solution in such a way that with each application (pressure) only a small controlled quantity may be provided to both nostrils.

While the examples have been described in detail in the foregoing description, the same is to be considered illustrative and not restrictive in character, being understood that only some examples have been shown and described and that all changes and modifications that come within the spirit of the examples are desired to be protected. While said particular examples of the present disclosure have been described, it would be obvious to those skilled in the art that various other changes and modifications may be made without departing from the scope of the appended claims.

### REFERENCES

**1.** Lirong Zou et al. SARS-CoV-2 Viral Load in Upper Respiratory Specimens of Infected Patients, N Engl J Med 2020; 382:1177-1179, DOI: 10.1056/NEJMc2001737.
**2.** Gengler I, Wang JC, Speth MM, Sedaghat AR. Sinonasal pathophysiology of SARS-CoV-2 and COVID-19: A systematic review of the current evidence. Laryngoscope Investigative Otolaryngology. 2020;1-6. https:// doi.org/10.1002/lio2.384.
**3.** Rafael R. G., Machado, Talita Glaser, Danielle B. Araujo, Lyvia Lintzmaier Petiz, Danielle B. L. Oliveira, Giuliana S. Durigon, Alessandra L. Leal, João Renato R. Pinho, Luis Carlos S. Ferreira, Henning Ulrich, Edison L. Durigon, Cristiane R. Guzzo , Hypertonic saline solution inhibits SARS-CoV-2 in vitro assay. BioRχiv, doi: https://doi.org/10.1101/2020.08.04.235549
**4.** Sandeep Ramalingam, Baiyi Cai, Junsheng Wong, Matthew Twomey, Rui Chen, Rebecca M. Fu, Toby Boote, Hugh McCaughan, Samantha J. Griffiths, Jürgen G. Haas, Antiviral innate immune response in non-myeloid cells is augmented by chloride ions via an increase in intracellular hypochlorous acid level, Scientific Reports (2018) 8:13630 | DOI:10.1038/s41598-018-31936-y (www.nature.com/scientificreports).
**5.** Sandeep Ramalingam, Catriona Graham, Jenny Dove, Lynn Morrice, Aziz Sheikh, A pilot, open labelled, randomised controlled trial of hypertonic saline nasal irrigation and gargling for the common cold, Scientific Reports (2019) 9:1015 https://doi.org/10.1038/s41598-018-37703-3 (www.nature.com/scientificreports).

## Claims

1. Disposable nasal liquid dispenser device for dispensing a controlled quantity of a liquid solution into the nasal cavities and / or paranasal sinuses of a user, comprising:
- a liquid solution holding cylinder (1) having a distal and a proximal end, said cylinder containing a quantity of liquid solution to be dispensed and comprising two cannulas (2) arranged at its distal end for dispensing the liquid solution into both nasal cavities simultaneously;
- two resilient nostril plugs (4) having a distal and proximal end, said nostril plugs (4) being designed to seal the nostrils upon application therein, and said nostril plugs (4) comprising a discharge orifice (5) at their distal end, and further comprising coupling means (6) configured to be mounted on said cannulas (2);
- a piston (7) comprising elastic piston seals to offer sealing on the liquid solution holding cylinder (1), wherein the piston is mounted in the cylinder (1) and designed to slide in an axial direction relative to said cylinder; and
- an actuator (8) mounted with the piston (7) and configured to move the piston along the inside of the cylinder (1) upon manual actuation of a user.

2. Disposable nasal dispenser device according to claim 1, wherein the nostril plugs (4) are designed to rest in an angle α1 of between 130° and 140°, when mounted on the cannulas (2).

3. Disposable nasal dispenser device according to claim 2, wherein the angle is 135°.

4. Disposable nasal dispenser device according to previous claims, wherein the nostril plugs (4) are designed to have a relative angle between them of between 15 and 20 degrees.

5. Disposable nasal dispenser device according to previous claims, wherein the nostril plugs (4) are affixed to the cylinder (1) by means of a snap-fit system (9).

6. Disposable nasal dispenser device according to previous claims, wherein the nostril plugs (4) are made from LDPE low density polyethylene.

7. Disposable nasal dispenser device according to previous claims, wherein the nostril plugs (4) are bell-shaped to lock into the nose.

8. Disposable nasal dispenser device according to previous claims, wherein the liquid solution holding cylinder (1) has two radially extending shoulders (3) arranged at its proximal end.

9. Disposable nasal dispenser device according to previous claims, wherein the actuator (8) detaches from the piston (7) when moved in an opposite direction with regard to the liquid solution holding cylinder (1), preventing the user from returning the piston to its original position.

10. Disposable nasal dispenser device according to previous claims, wherein the actuator (8) and the cylinder (1) are provided with co-operating guiding features extending in the direction of actuation.

11. Disposable nasal dispenser device according to previous claims, wherein the actuator (8) has an actuation surface (10) configured to move the piston along the inside of the cylinder (1) upon manual actuation of a user on the same.

12. Disposable nasal dispenser device according to previous claims, wherein the liquid solution is a saline solution.

13. Disposable nasal dispenser device according to previous claims, wherein the liquid solution is a medicinal solution.

14. Disposable nasal dispenser device according to previous claims, **characterized in that** the bell-shape and angle configuration of the nostril plugs (4) generates an airtight seal between the plugs and the nostrils causing the soft palate to elevate, sealing off of the nasal cavity, and allowing the liquid solution to reach the paranasal sinuses.

## Patentansprüche

1. Einweg-Nasenflüssigkeitsabgabevorrichtung zur Abgabe einer kontrollierten Menge einer Flüssigkeitslösung in die Nasenhöhlen und/oder Nasennebenhöhlen eines Benutzers, die Folgendes umfasst:
- einen Flüssigkeitslösungsaufnahmezylinder (1), der ein distales und ein proximales Ende aufweist, wobei der Zylinder eine abzugebende Flüssigkeitslösungsmenge enthält und zwei an seinem distalen Ende angeordnete Kanülen (2) zur gleichzeitigen Abgabe der Flüssigkeitslösung in beide Nasenhöhlen umfasst;
- zwei elastische Nasenlochstöpsel (4), die ein distales und ein proximales Ende aufweisen, wobei die Nasenlochstöpsel (4) so gestaltet sind, dass sie die Nasenlöcher bei Anwendung darin abdichten, und die Nasenlochstöpsel (4) eine Auslassöffnung (5) an ihrem distalen Ende umfassen und ferner Kopplungsmittel (6) umfassen, die so konfiguriert sind, dass sie an den Kanülen (2) angebracht werden können;
- einen Kolben (7), der elastische Kolbendichtungen umfasst, um eine Abdichtung auf dem Flüssigkeitslösungsaufnahmezylinder (1) zu bieten, wobei der Kolben in dem Zylinder (1) angebracht ist und so gestaltet ist, dass er in einer axialen Richtung relativ zu dem Zylinder gleitet; und
- ein Betätigungselement (8), das mit dem Kolben (7) angebracht ist und so konfiguriert ist, dass es den Kolben bei manueller Betätigung durch einen Benutzer entlang der Innenseite des Zylinders (1) bewegt.

2. Einweg-Nasenflüssigkeitsabgabevorrichtung nach Anspruch 1, wobei die Nasenlochstöpsel (4) so gestaltet sind, dass sie sich in einem Winkel α1 zwischen 130° und 140° befinden, wenn sie an den Kanülen (2) angebracht sind.

3. Einweg-Nasenflüssigkeitsabgabevorrichtung nach Anspruch 2, wobei der Winkel 135° beträgt.

4. Einweg-Nasenflüssigkeitsabgabevorrichtung nach einem der vorhergehenden Ansprüche, wobei die Nasenlochstöpsel (4) so gestaltet sind, dass sie einen relativen Winkel von 15 bis 20 Grad zwischen sich aufweisen.

5. Einweg-Nasenflüssigkeitsabgabevorrichtung nach einem der vorhergehenden Ansprüche, wobei die Nasenlochstöpsel (4) mittels eines Einschnappsystems (9) an dem Zylinder (1) befestigt sind.

6. Einweg-Nasenflüssigkeitsabgabevorrichtung nach einem der vorhergehenden Ansprüche, wobei die Nasenlochstöpsel (4) aus LDPE-Polyethylen niedriger Dichte hergestellt sind.

7. Einweg-Nasenflüssigkeitsabgabevorrichtung nach einem der vorhergehenden Ansprüche, wobei die Nasenlochstöpsel (4) glockenförmig sind, um in die Nase einzurasten.

8. Einweg-Nasenflüssigkeitsabgabevorrichtung nach einem der vorhergehenden Ansprüche, wobei der Flüssigkeitslösungsaufnahmezylinder (1) zwei sich radial erstreckende Schultern (3) aufweist, die an seinem proximalen Ende angeordnet sind.

9. Einweg-Nasenflüssigkeitsabgabevorrichtung nach einem der vorhergehenden Ansprüche, wobei sich das Betätigungselement (8) von dem Kolben (7) löst, wenn es in eine entgegengesetzte Richtung in Bezug auf den Flüssigkeitslösungsaufnahmezylinder (1) bewegt wird, was verhindert, dass der Benutzer den Kolben in seine ursprüngliche Position zurücksetzt.

10. Einweg-Nasenflüssigkeitsabgabevorrichtung nach einem der vorhergehenden Ansprüche, wobei das Betätigungselement (8) und der Zylinder (1) mit zusammenwirkenden, sich in die Betätigungsrichtung erstreckenden Führungselementen versehen sind.

11. Einweg-Nasenflüssigkeitsabgabevorrichtung nach einem der vorhergehenden Ansprüche, wobei das Betätigungselement (8) eine Betätigungsoberfläche (10) aufweist, die so konfiguriert ist, dass sie den Kolben bei manueller Betätigung desselben durch einen Benutzer entlang der Innenseite des Zylinders (1) bewegt.

12. Einweg-Nasenflüssigkeitsabgabevorrichtung nach einem der vorhergehenden Ansprüche, wobei die Flüssigkeitslösung eine Kochsalzlösung ist.

13. Einweg-Nasenflüssigkeitsabgabevorrichtung nach einem der vorhergehenden Ansprüche, wobei die Flüssigkeitslösung eine medizinische Lösung ist.

14. Einweg-Nasenflüssigkeitsabgabevorrichtung nach einem der vorhergehenden Ansprüche, **dadurch gekennzeichnet, dass** die glockenförmige und gewinkelte Konfiguration der Nasenlochstöpsel (4) eine luftdichte Abdichtung zwischen den Stöpseln und den Nasenlöchern erzeugt, wodurch sich das Gaumensegel hebt, die Nasenhöhle abgedichtet wird und die Flüssigkeitslösung in die Nasennebenhöhlen gelangen kann.

## Revendications

1. Dispositif de distribution de liquide nasal jetable destiné à distribuer une quantité contrôlée d'une solution liquide dans les cavités nasales et/ou les sinus paranasaux d'un utilisateur, comprenant :
- un cylindre de maintien de solution liquide (1) ayant une extrémité distale et une extrémité proximale, ledit cylindre contenant une quantité de solution liquide à distribuer et comprenant deux canules (2) agencées au niveau de son extrémité distale pour distribuer la solution liquide dans les deux cavités nasales simultanément ;
- deux bouchons de narine souples (4) ayant une extrémité distale et une extrémité proximale, lesdits bouchons de narine (4) étant conçus pour sceller les narines lors de leur application dans celles-ci, et lesdits bouchons de narine (4) comprenant un orifice de décharge (5) au niveau de leur extrémité distale, et comprenant en outre des moyens de couplage (6) configurés pour être montés sur lesdites canules (2) ;
- un piston (7) comprenant des joints de piston élastiques pour assurer un scellement sur le cylindre de maintien de solution liquide (1), dans lequel le piston est monté dans le cylindre (1) et conçu pour glisser dans une direction axiale par rapport audit cylindre ; et
- un actionneur (8) monté avec le piston (7) et configuré pour déplacer le piston le long de l'intérieur du cylindre (1) lors de l'actionnement manuel d'un utilisateur.

2. Dispositif de distribution nasale jetable selon la revendication 1, dans lequel les bouchons de narine (4) sont conçus pour reposer à un angle α1 compris entre 130° et 140°, lorsqu'ils sont montés sur les canules (2).

3. Dispositif de distribution nasale jetable selon la revendication 2, dans lequel l'angle est de 135°.

4. Dispositif de distribution nasale jetable selon les revendications précédentes, dans lequel les bouchons de narine (4) sont conçus pour avoir un angle relatif entre eux compris entre 15 et 20 degrés.

5. Dispositif de distribution nasale jetable selon les revendications précédentes, dans lequel les bouchons de narine (4) sont fixés au cylindre (1) au moyen d'un système d'encliquetage (9).

6. Dispositif de distribution nasale jetable selon les revendications précédentes, dans lequel les bouchons de narine (4) sont fabriqués en polyéthylène basse densité LDPE.

7. Dispositif de distribution nasale jetable selon les revendications précédentes, dans lequel les bouchons de narine (4) sont en forme de cloche pour se bloquer dans le nez.

8. Dispositif de distribution nasale jetable selon les revendications précédentes, dans lequel le cylindre de maintien de solution liquide (1) a deux épaulements (3) s'étendant radialement agencés au niveau de son extrémité proximale.

9. Dispositif de distribution nasale jetable selon les revendications précédentes, dans lequel l'actionneur (8) se détache du piston (7) lorsqu'il est déplacé dans une direction opposée par rapport au cylindre de maintien de solution liquide (1), empêchant l'utilisateur de remettre le piston dans sa position d'origine.

10. Dispositif de distribution nasale jetable selon les revendications précédentes, dans lequel l'actionneur (8) et le cylindre (1) sont pourvus d'éléments de guidage coopératifs s'étendant dans la direction d'actionnement.

11. Dispositif de distribution nasale jetable selon les revendications précédentes, dans lequel l'actionneur (8) a une surface d'actionnement (10) configurée pour déplacer le piston le long de l'intérieur du cylindre (1) lors de l'actionnement manuel d'un utilisateur sur celui-ci.

12. Dispositif de distribution nasale jetable selon les revendications précédentes, dans lequel la solution liquide est une solution saline.

13. Dispositif de distribution nasale jetable selon les revendications précédentes, dans lequel la solution liquide est une solution médicinale.

14. Dispositif de distribution nasale jetable selon les revendications précédentes, **caractérisé en ce que** la configuration en forme de cloche et en angle des bouchons de narine (4) génère un joint étanche à l'air entre les bouchons et les narines amenant le palais mou à se soulever, scellant la cavité nasale, et permettant à la solution liquide d'atteindre les sinus paranasaux.
